# FASCICULE DE BREVET EUROPEEN

(11) **EP 1 536 764 B1**
(45) Date de publication et mention de la délivrance du brevet: **09.04.2008**
(21) Numéro de dépôt: 03762747.8
(22) Date de dépôt: 08.07.2003
(51) Int. Cl.: A61Q 19/00

(54) **COMPOSITION COSMETIQUE CAPABLE DE LUTTER CONTRE LE VIEILLISSEMENT CUTANE**
KOSMETISCHE ZUSAMMENSETZUNG GEGEN DAS ALTERN DER HAUT
COSMETIC COMPOSITION FOR FIGHTING AGAINST SKIN AGEING

(30) Priorité: 08.07.2002 FR 0208564
(43) Date de publication de la demande: 08.06.2005
(73) Titulaire: Laboratoires Clarins, 92200 Neuilly sur Seine (FR)
(72) Inventeur: COURTIN, Olivier, F-92100 Boulogne-Billancourt (FR)
(74) Mandataire: Breese, Pierre
(86) Numéro de dépôt international: PCT/FR2003/002124
(87) Numéro de publication internationale: WO 2004/004680

(56) Documents cités:
- DATABASE WPI Section Ch, Week 200319 Derwent Publications Ltd., London, GB; Class B04, AN 2003-195566 XP002265125 & KR 2002 081 995 A (DRIGO CO LTD), 30 octobre 2002 (2002-10-30)
- DATABASE WPI Section Ch, Week 200234 Derwent Publications Ltd., London, GB; Class D13, AN 2002-292944 XP002265126 & CN 1 334 024 A (SCI & TECHONLOGY DEV GEN CO YUNNAN PROV), 6 février 2002 (2002-02-06)
- DATABASE WPI Section Ch, Week 200203 Derwent Publications Ltd., London, GB; Class B04, AN 2002-018128 XP002265127 & CN 1 308 879 A (XUE W), 22 août 2001 (2001-08-22)
- DATABASE WPI Section Ch, Week 200065 Derwent Publications Ltd., London, GB; Class B04, AN 2000-665687 XP002265128 & CN 1 265 287 A (SHI Y), 6 septembre 2000 (2000-09-06)
- DATABASE WPI Section Ch, Week 199715 Derwent Publications Ltd., London, GB; Class B04, AN 1997-155029 XP002265129 & CN 1 091 249 A (XUE Z), 31 août 1994 (1994-08-31)

## Description

La présente invention concerne une nouvelle composition cosmétique capable de lutter contre le vieillissement cutané contenant deux extraits végétaux : *Diospyros kaki* et *Pueraria lobata*.

**Abrégé de la demande de brevet** CN 1 334 024 **XP002265126, décrit un ingrédient alimentaire riche en iode préparé à partir de Diospyros kaki, de racine de Pueraria, d'igname et de rhizome de lis.**

Par "composition cosmétique capable de lutter contre le vieillissement cutané" on entend toute composition cosmétique capable d'empêcher ou de limiter les conséquences de vieillissement chronologique et/ou actinique au niveau du collagène cutané et par voie de conséquence d'empêcher ou de limiter l'apparition cutanée des signes visibles du vieillissement de la surface de la peau : les rides et les ridules.

Le vieillissement cutané se caractérise entre autre par une diminution globale de l'épaisseur de la peau (perte de 6 % dès la naissance tous les dix ans), une diminution du renouvellement cellulaire, une diminution du collagène au niveau du derme, associé à une augmentation de la glycation des protéines cutanées.

A ce processus chronologique, s'ajoute, pour les femmes, un vieillissement que l'on peut qualifier de vieillissement hormonal, consécutif à la chute de la production d'oestrogènes au moment de la ménopause. Cette déficience en oestrogènes affecte au niveau dermique les fibroblastes, et par conséquent tous les composants de la matrice extra-cellulaire, le collagène en particulier.

La glycation non enzymatique des protéines représente la première cause de modification du collagène cutané. Il existe différents types de collagène qui représentent 70 à 80 % du poids sec du derme. Ces collagènes sont constitués d'une triple hélice caractéristique et sont organisés, en fibres. Des pontages naturels enzymo-dépendants stabilisent normalement les fibrilles de collagène. Mais le phénomène de glycation peut, lui aussi, être responsable d'un autre type de pontage dû à une fixation non enzymatique du glucose. Les fibres de collagène deviennent alors plus rigides, plus résistantes à la pression, et plus résistantes aux collagénases, le renouvellement du collagène n'est plus assuré, les tissus sont altérés, rigides et cassants. Il s'agit d'un véritable processus de réticulation avec la création de liaisons croisées anormales et irréversibles. Cette réticulation est une des expressions majeures du vieillissement cutané.

Les collagènes glyqués ainsi pontés perdent leurs fonctionnalité biologique ; les tissus se rigidifient et se sclérosent. La glycation dépend des possibilités de rencontre entre les molécules de glucose circulantes et les groupements aminés libres existants soit à l'extrémité N-terminale des chaînes polypeptidiques, soit sur les chaînes latérales de lysine.

Le mécanisme biochimique de cette réaction est bien connu et comporte deux phases :
1- Une phase précoce (initiation) : réaction de sucres réducteurs (glucose, fructose) avec les fonctions amines terminales ou latérales des protéines constitutives tissulaires pour produire des composés appelés "Bases de Schiff". Ces composés sont stabilisés ensuite par réarrangement d'Amadori en cétoamine.
2- Une phase tardive (propagation et terminaison) : les fonctions cétoamine sont oxydées en désoxyosone en présence d'oxygène et réagissent avec d'autres acides aminés basiques tels que l'arginine ou la lysine appartenant à d'autres protéines (albumine, lipoprotéines, immunoglobulines). Le résultat est la formation de complexes avec des pontages finaux du type cycles pentosidine ou furoyl 6.3. furonyl 1.4 imidazole.

L'une des conséquences importantes de la glycation des protéines est la création de radicaux libres. En effet, lorsqu'elles sont glyquées, les protéines réagissent avec l'oxygène et provoquent la formation de radicaux type superoxyde. Ces derniers sont capables d'initier la dégradation de protéines, d'altérer les structures membranaires, désorganisant au final la matrice extra-cellulaire ainsi que tous ses composants.

Le collagène cutané, ainsi que tous les autres composants de la matrice extra-cellulaire, est aussi affecté par la diminution des oestrogènes au moment de la ménopause. En effet, la chute du taux d'oestrogènes provoque la diminution de la synthèse du collagène par les fibroblastes ainsi qu'une augmentation de sa dégradation. Cet effondrement est rapide et peut atteindre 30 % dans les cinq premières années de la ménopause. La production de fibres de type III, plus fines, contribue à la perte annuelle de 1 % en épaisseur du derme. Enfin, il existe aussi une altération des glycoprotéines de structure ayant pour conséquence la perturbation de l'organisation tridimensionnelle de la matrice extracellulaire.

Ainsi, une composition cosmétique utile pour lutter contre le vieillissement cutané doit contenir des actifs capables de freiner la glycation des protéines et de combler le déficit en oestrogènes. On protègera ainsi le collagène, molécule majeure de la peau qui lui confère ses propriétés mécaniques. L'apparition des rides, la diminution de tonicité cutanée étant le reflet du vieillissement du derme et notamment de ses propriétés mécaniques (diminution quantitative du réseau de collagène, tout comme sa rigidification) .

Les travaux de la Demanderesse ont permis de mettre en évidence que l'association de deux extraits végétaux de *Diospyros kaki* et de *Pueraria lobata* présentait une action protectrice du collagène cutané, respectivement une activité anti-glycation et une activité "hormone-like", qui font de ces deux extraits une composition utile pour lutter contre le vieillissement cutané.

L'invention concerne, tout particulièrement, une composition contenant un extrait de *Diospyros kaki* et un extrait de *Pueraria lobata*.

Le *Diospyros kaki* appelé aussi plaqueminier est un arbre originaire des régions chaudes et principalement d'Indonésie. Il peut se cultiver dans les régions où poussent les oliviers et les figuiers. Les feuilles caduques laissent apparaître, après leur chute, des fruits jaunes puis oranges. Des usages locaux mettent en évidence les propriétés de différentes parties de l'arbre : l'écorce des tiges est astringente ; le fruit est utilisé en médecine traditionnelle pour ses propriétés anti-tussive, laxative et nutritive, le jus de fruit frais est utilisé contre l'hypertension...

La composition cosmétique, objet de la présente invention contient un extrait de *Diospyros kaki.*

De façon avantageuse, cet extrait est un extrait issu de calices de *Diospyros kaki*. De préférence il s'agit d'un extrait hydrosoluble et, plus particulièrement, un extrait hydroglycolique. Cet extrait est avantageusement obtenu selon le protocole suivant :
(i) solubilisation des calices de *Diospyros kaki* dans le butylène glycol,
(ii) séparation des phases soluble et insoluble par filtration,
(iii) filtration stérilisante.

L'extrait hydrosoluble et plus particulièrement hydroglycolique de *Diospyros kaki* utilisé dans la composition selon l'invention, a un aspect liquide limpide, une couleur ambrée et une odeur caractéristique. Il présente les caractéristiques analytiques suivante
- matières sèches = 9-16 g/l.
- polyphénols totaux = 2-4 g/l (flavanol, acide hydroxycinnamique, acide protocatéchique et acide abscissique).
- pH = 5-6.

*Pueraria lobata* appelé aussi Kudzu est une liane ligneuse de la famille des légumineuses, originaire d'Asie méridionale et tropicale. Cette plante porte des fleurs pourpres très parfumées retombant en longues grappes et produisant des gousses brunes renfermant 3 à 10 graines à épais tégument. Ses racines sont épaisses ou tubéreuses, ses feuilles alternées sont recouvertes d'un long duvet brun très dense. *Pueraria lobata* contient des molécules naturelles qui ont une activité similaire à celle des oestrogènes. Ces substances non stéroïdiennes sont des isoflavones telles que la génistéine, la daidzéine, le coumestrol et la pratenséine.

La composition cosmétique objet de la présente invention contient un extrait de *Pueraria lobata*, avantageusement issu des racines de la plante. De préférence, il s'agit d'un extrait hydrosoluble et, plus particulièrement, hydroglycolique. Cet extrait est avantageusement obtenu selon le protocole suivant :
(a) broyage à 2 mm, 1 unité poids,
(b) ajout d'éthanol 5 unités volume,
(c) extraction à reflux pendant 4 heures,
(d) filtration-concentration-évaporation à sec puis ajout de glycérine-eau.
(e) filtration à 0,2 µm.

L'extrait hydrosoluble et plus particulièrement hydroglycolique de *Pueraria lobata* utilisé dans la composition selon l'invention, a un aspect liquide limpide de couleur jaune orangé et d'odeur caractéristique. Il présente les caractéristiques analytiques suivantes :
- Indice de réfraction (à 25°C) = 1,385-1,405
- Densité (à 20°C) = 1,0-1,2
- Teneur en isoflavones (HPLC) = 0,3-0,6 %
- pH = 4,5-6,5.

La composition selon l'invention contient :
- de l'ordre de 0 , 1 à 10 % en poids et de préférence 0,5 à 5 % en poids d'extrait de *Diospyros kaki* et
- de l'ordre de 0,1 à 10 % en poids et préférence 0,5 à 5 % en poids d'extrait de *Pueraria lobata*.

La composition objet de la présente invention peut également contenir tout extrait végétal riche en flavonoïdes et/ou en tanins. En effet, les flavonoïdes interagissent avec les fibres de collagène et les protègent très efficacement de la dégradation protéolytique présente lors du vieillissement cutané. De plus, la richesse en tanins des extraits végétaux leur confèrent des activités stimulante, anti-oxydante et protectrice. Une activité anti-oxydante est particulièrement utile pour lutter contre le vieillissement cutané. A titre d'exemple et de façon non exhaustive, on peut citer comme extrait végétal riche en tanins utilisable dans le cadre de la présente invention un extrait de cardère sauvage (*Dipscacus sylvestris*). Le cardère sauvage également appelé "Peigne-de-loup", "Baignoire de Vénus", ou "Cabaret-des-oiseaux" est une plante herbacée bisannuelle de 60 à 150 cm que l'on trouve partout en Europe au bord des chemins et des fossés.

L'extrait de cardère sauvage utilisable dans le cadre de l'invention est, plus particulièrement, obtenu par digestion dans un mélange hydroglycolique à partir de la partie aérienne de la plante finement broyée, puis il est décoloré, clarifié, standardisé et conservé.

Les extraits végétaux riches en flavonoïdes utilisables sont avantageusement choisis dans le groupe constitué par un extrait de romarin (*Rosmarinus officinalis*), un extrait de galanga (*Alpinia officinarum*) et un extrait de buddleja (*Buddleja davidii*).

Un extrait de romarin (Rosmarinus officinalis) utilisable dans le cadre de l'invention est avantageusement un extrait hydrosoluble obtenu à partir des feuilles de cette plante commune de la région méditerranéenne. Cet extrait contient des flavonoïdes, de l'acide ursolique et de l'acide rosmarinique et est obtenu selon le procédé décrit dans la demande de brevet français publiée sous le numéro FR9815394.

Le galanga (*Alpinia officinarum*) de la famille des Zingibéracées est un arbrisseau originaire de l'Est de l'Asie dont le rhizome noir et noueux ressemble à celui du gingembre. L'extrait de galanga utilisable dans la composition de l'invention est un extrait hydrosoluble de rhizomes de galanga obtenu par extraction contrôlée par le mélange eau/ propylène glycol suivie d'une étape de centrifugation/ filtration. Cet extrait de galanga est riche en phytostérols et flavonoïdes (1,5 g/l équivalent rutine) tels que la galangine et le kaempférol et en huiles essentielles comme le bornéol, le cinéole, le caryophyllène et le géraniol.

L'extrait de buddleja (*Buddleja davidii*) utilisable dans le cadre de la présente invention est avantageusement un extrait hydrosoluble de buddleja obtenu à partir de plantes séchées et plus particulièrement à partir de fleurs et de feuilles séchées. Les fleurs et feuilles séchées sont broyées puis une extraction éthanol/eau est réalisée. L'extrait ainsi obtenu est concentré après une étape de filtration et, éventuellement, une étape de formulation dans la glycérine. L'extrait hydrosoluble de buddleja utilisable dans la composition de l'invention contient des iridoïdes, des flavonoïdes (agents anti-oxydants), des esters de l'acide caféique (agents anti-oxydants) et des triterpénoïdes.

Les compositions selon l'invention peuvent encore comprendre un ou plusieurs agents de formulation ou additifs d'usage connu et classique dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc... Grâce à ces connaissances en matière de cosmétique, l'homme du métier saura quels agents de formulation ajouter aux compositions de l'invention et en quelles quantités en fonction des propriétés recherchées.

Il faut remarquer que l'ajout de filtres UV dans la composition cosmétique selon l'invention permet de lutter contre le vieillissement actinique induit par les UV. Les filtres UV utilisables dans la composition cosmétique selon l'invention peuvent être des filtres chimiques ou minéraux. De façon avantageuse, les filtres UV utilisables dans la composition cosmétique selon l'invention sont les filtres décrits dans la demande de brevet français publiée sous le numéro FR2811224.

Les compositions selon l'invention peuvent se présenter sous toute forme connue de l'homme du métier dans le domaine de cosmétologie et de la dermatologie sans autre restriction galénique que l'application sur le visage et sur le corps. De façon avantageuse, les compositions selon l'invention se présentent sous la forme d'un gel, d'une lotion, d'une crème, d'une émulsion, d'un lait, d'un lait, d'un spray, etc...

La présente invention concerne également l'utilisation d'une composition selon l'invention pour prévenir le vieillissement cutané et/ou lutter contre ce denier.

La présente invention concerne également l'utilisation d'extraits de *Diospyros kaki* et de *Pueraria lobata*, pour la préparation d'une composition cosmétique ou dermatologique pour prévenir le vieillissement cutané et/ou lutter contre ce denier.

D'autres avantages et caractéristiques de l'invention apparaîtront à la lecture des exemples qui suivent et qui concernent l'effet de l'extrait de *Diospyros kaki* et de *Pueraria lobata* sur le collagène cutané, et des exemples de formulation qui sont donnés à titre illustratif et ne sauraient être interprétés comme limitant la portée de l'invention.

### I - Effet de l'extrait de Diospyros kaki sur la glycation des protéines.

L'effet de l'extrait de *Diospyros kaki* a été étudié *in-vitro* sur un modèle qui repose sur la mesure de la formation des dérivés de la réaction de la glycation entre la lysine et le glucose 6-phosphate. Certains de ces dérivés (AGE) sont fluorescents. La réaction a été mesurée après 15 jours d'incubation.

### Mode opératoire :

Une molécule de référence a été testée en parallèle : l'aminoguanidine.

### Système d'essai (SE):

Le système d'essai est le mélange réactionnel contenant l'albumine bovine (0,5 g/ml) et le glucose (500 mM) dans un tampon phosphate (0,2 M, pH = 7,4).

### Incubation produits + SE :

Les produits sont mélangés au système d'essai dans des tubes stériles ; ces derniers sont recouverts de papier aluminium (la réaction doit se faire à l'abri de la lumière).

Pour les conditions expérimentales ou certains réactifs ou produits ne seront pas présents, il faut compléter le volume du tube QSP avec de l'eau MilliQ.

Après avoir fermé l'ouverture des tubes avec du parafilm (la réaction doit se dérouler à l'abri de l'oxygène), les différents mélanges réactionnels sont placés dans une étuve sèche à 37°C pendant 8 jours.

### Evaluation des effets :

Après les 8 jours d'incubation, 100 µl de chaque tube est prélevé et transféré dans une plaque noire 96 puits.
La fluorescence est alors lue au FLUOstar (*BMG*) (excitation à 355 nm, émission à 460 nm). Les résultats seront exprimés en unité arbitraire de fluorescence.

La fluorescence relative aux échantillons « produit + albumine bovine » (= interférence) sera soustraite des données « produit + albumine bovine + Glucose ».

### Résultats :

Après 8 jours d'incubation d'albumine bovine avec une surcharge en glucose, la formation de « cross - linking » de glycation est observée. Elle a été évaluée en tirant parti des propriétés de fluorescence de l'adduit albumine bovine-glucose.
L'extrait de kaki présent dans le système d'essai empêche la liaison croisée entre l'albumine bovine et le glucose de manière assez efficacement (voir tableau 1 ci-dessous).

**Tableau 1 : Activité anti-glycation de l'extrait de Diospyros kaki.**

| Produit | Intensité de Fluorescence (unité arbitraire) | Interférence (sans glucose) (unité arbitraire) | Intensité de Fluorescence Réelle (unité arbitraire) | % d'inhibition de la glycation |
|---|---|---|---|---|
| Témoin (BSA + Glucose) | 50229,5 | 291 | 49938,5 | / |
| Aminoguanidine (10 mM) | 13011,5 | 17537 | 0 | 100 |
| Diospyros kaki 0,5 % | 34131,5 | 21093,5 | 13038 | 73,9 |
| Diospyros kaki 1% | 34282 | 24883 | 9399 | 81,2 |
| Diospyros kaki 3% | 9869 | 33893 | 5976 | 88,0 |
| Diospyros kaki 5% | 43052,5 | 37550,5 | 5502 | 89,0 |

### II - Effet de l'extrait de Pueraria lobata sur la synthèse des protéines.

L'effet de l'extrait de Pueraria lobata a été étudié in-vitro sur culture de fibroblastes : mesure de la stimulation du métabolisme cellulaire et de la synthèse des protéines.

### Principe :

Des fibroblastes humains normaux sont cultivés dans un milieu DMEM classique supplémenté en sérum de veau foetal en présence d'extrait de *Pueraria lobata*. Après un temps d'incubation optimal, le nombre de cellules est compté sous microscope, la quantité totale de protéines intra- et extracellulaires dans le milieu est déterminée selon la méthode BCA. Le métabolisme cellulaire est quantifié par la méthode MTT qui indique le taux de respiration mitochondriale.

### Résultats :

L'extrait de *Pueraria lobata* stimule le métabolisme et la synthèse de protéines d'une manière dose dépendante.
Les valeurs MTT (respiration cellulaire mitochondriale) et la quantité de protéines par 1.000 cellules sont déterminées parallèlement mais indépendamment sur la même culture de cellules. L'extrait de *Pueraria lobata* à 0,3 % augmente de 44 % la conversion du MTT. A 1 % d'extrait de *Pueraria lobata*, cette augmentation atteint 66 %. Pour le même temps, la quantité totale de protéines synthétisées par les cellules atteint 99 %.

### III - Exemples de formulations selon l'invention.

### CRÈME

| | |
|---|---|
| EMULSIONNANTS | 7,000 |
| TRIGLYCÉRIDES | 10,00 |
| BEURRE DE KARITÉ | 2,000 |
| HUILES DE SILICONE | 1,500 |
| AGENT GÉLIFIANT NEUTRALISÉ | 0,600 |
| GLYCÉRINE | 5,000 |
| AGENT SÉQUESTRANT | 0,100 |
| EXTRAIT DE CARDÈRE | 2,000 |
| EXTRAIT DE BUDDLEJA | 1,000 |
| EXTRAIT DE ROMARIN | 1,000 |
| EXTRAIT DE KAKI | 3,000 |
| EXTRAIT DE PUERARIA | 3,000 |
| HUILE DE PEQUI | 2,000 |
| PARFUM | 0,500 |
| CONSERVATEURS | 1,000 |
| EAU PURIFIÉE | Q.S.P. 100 |

### GEL

| | |
|---|---|
| BUTYLÈNE GLYCOL | 2,000 |
| AGENT ÉPAISSISSANT NEUTRALISÉ | 1,000 |
| AGENT SÉQUESTRANT | 0,100 |
| ALCOOL ÉTHYLIQUE | 5,000 |
| EXTRAIT DE KAKI | 3,000 |
| EXTRAIT DE PUERARIA | 3,000 |
| EXTRAIT DE CARDÈRE | 2,000 |
| EXTRAIT DE ROMARIN | 2,000 |
| SOLUBILISANT | 1,000 |
| PARFUM | 0,500 |
| CONSERVATEURS | 0,700 |
| EAU PURIFIÉE | Q.S.P. 100 |

### SÉRUM

| | |
|---|---|
| AGENT GÉLIFIANT | 0,600 |
| GLYCÉRINE | 2,000 |
| BUTYLÈNE GLYCOL | 3,000 |
| AGENT SÉQUESTRANT | 0,100 |
| EXTRAIT DE ROMARIN | 1,000 |
| EXTRAIT DE CARDÈRE | 2,000 |
| EXTRAIT DE BUDDLEJA | 2,000 |
| EXTRAIT DE GALANGA | 1,000 |
| EXTRAIT DE KAKI | 4,000 |
| EXTRAIT DE PUERARIA | 4,000 |
| TRIGLYCÉRIDES | 2,000 |
| HUILES DE SILICONE | 7,000 |
| CONSERVATEURS | 0,600 |
| PARFUMS | 0,400 |
| EAU PURIFIÉE | Q.S.P. 100 |

## Revendications

1. Composition cosmétique **caractérisée en ce qu'**elle contient :
- un extrait de *Diospyros kaki* et,
- un extrait de *Pueraria lobata,*
- et un ou plusieurs agents de formulation ou additifs d'usage connu et classique dans les compositions cosmétiques et dermatologiques.

2. Composition cosmétique, selon la revendication 1, **caractérisée en ce que** l'extrait de *Diospyros kaki* est un extrait issu de calices de la plante et dont la concentration en polyphénols totaux est de 2 à 4 g/l.

3. Composition cosmétique selon l'une quelconque des revendications 1 ou 2, **caractérisée en ce que** l'extrait de *Pueraria lobata* est un extrait issu des racines de la plante et dont la teneur en isoflavones est de 0,3 à 0,6 %.

4. Composition cosmétique selon l'une quelconque des revendications 1 à 3, **caractérisée en ce qu'**elle contient :
- de l'ordre de 0 , 1 à 10 % en poids et de préférence 0,5 à 5 % en poids d'extrait de *Diospyros kaki.*
- de l'ordre de 0,1 à 10 % en poids et de préférence 0,5 à 5 % en poids d'extrait de *Pueraria lobata*.

5. Composition cosmétique selon l'une quelconque des revendications 1 à 4, **caractérisée en ce qu'**elle contient en outre au moins un actif végétal riche en flavonoïdes et/ou en tanins choisi dans le groupe constitué par un extrait de cardère sauvage (*Dipscacus sylvestris*), un extrait de romarin (*Rosmarinus officinalis*), un extrait de galanga (*Alpina officinarum*), un extrait de buddleja (*Buddleja davidii*).

6. Composition cosmétique selon l'une quelconque des revendications précédentes **caractérisée en ce qu'**elle comprend en outre un ou plusieurs agents de formulation ou additifs tels que des adoucissants, des colorants, des agents filmogènes, des tensio-actifs, des parfums, des conservateurs, des huiles, des glycols, des vitamines et des filtres UV.

7. Utilisation d'une composition cosmétique selon les revendications 1 à 6, pour prévenir le vieilissement cutané et/ou lutter contre ce denier.

8. Utilisation d'extraits de *Diospyros kaki* et de *Pueraria lobata* comme définis selon l'une quelconque des revendications 1 à 4, pour la préparation d'une composition cosmétique ou dermatologique pour prévenir le vieillissement cutané et/ou lutter contre ce denier.

## Claims

1. Cosmetic composition, **characterized in that** it comprises:
- an extract of *Diospyros kaki* and
- an extract of *Pueraria lobata*,
- and one or more formulation agents or additives of known and conventional use in cosmetic and dermatological compositions.

2. Cosmetic composition according to claim 1, **characterized in that** the extract of *Diospyros kaki* is an extract derived from the calyxes of the plant and the total polyphenol concentration of which is from 2 to 4 g/l.

3. Cosmetic composition according to any one of claim 1 or 2, **characterized in that** the extract of *Pueraria lobata* is an extract derived from the roots of the plant and the total isoflavone content of which is from 0.3 to 0.6%.

4. Cosmetic composition according to any one of claims 1 to 3, **characterized in that** it contains:
- on the order of from 0.1 to 10% by weight, preferably 0.5 to 5% by weight, of *Diospyros kaki* extract
- on the order of from 0.1 to 10% by weight, preferably 0.5 to 5% by weight, of *Pueraria lobata* extract.

5. Cosmetic composition according to any one of claims 1 to 4, **characterized in that** it further comprises at least one active plant ingredient rich in flavonoids and/or in tannins selected from among the groups consisting of an extract of wild teasel (*Dipsacus sylvestris*), an extract of rosemary (*Rosmarinus officinalis*), an extract of galangal (*Alpina officinarum*) and an extract of buddleia (*Buddleia davidii*).

6. Cosmetic composition according to any one of the preceding claims **characterized in that** it further comprises one or more formulation agents or additives such as softeners, coloring agents, film-forming agents, surface-active agents,
perfumes, preservatives, oils, glycols, vitamins and UV filters.

7. Use of a cosmetic composition according to claims 1 to 6 to prevent skin aging and/or to fight against it.

8. Use of extracts of *Diospyros kaki* and of *Pueraria lobata* as defined according to any one of claims 1 to 4 for the preparation of a cosmetic or dermatological composition for preventing skin aging and/or fighting against it.

## Patentansprüche

1. Kosmetische Zusammensetzung, **dadurch gekennzeichnet, dass** sie enthält:
- einen Auszug aus *Diospyros kaki* und
- einen Auszug aus *Pueraria lobata,*
- und eine oder mehrere Formulierungssubstanzen oder Zusatzstoffe, deren Verwendung in den kosmetischen und dermatologischen Zusammensetzungen bekannt und klassisch ist.

2. Kosmetische Zusammensetzung nach Anspruch 1, **dadurch gekennzeichnet, dass** der Auszug aus *Diospyros kaki* ein Auszug ist, der aus den Blütenkelchen der Pflanze stammt und dessen Gesamt-Polyphenolkonzentration von 2 bis 4 g/l beträgt.

3. Kosmetische Zusammensetzung nach einem der Ansprüche 1 oder 2, **dadurch gekennzeichnet, dass** der Auszug aus *Pueraria lobata* ein Auszug ist, der aus den Wurzeln der Pflanze stammt und dessen Isoflavon-Gehalt von 0,3 bis 0,6 % beträgt.

4. Kosmetische Zusammensetzung nach einem der Ansprüche 1 bis 3, **dadurch gekennzeichnet, dass** sie enthält:
- Auszug aus *Diospyros kaki* in der Größenordnung von 0,1 bis 10 Gew.-% und vorzugsweise 0,5 bis 5 Gew.-%,
- Auszug aus *Pueraria lobata* in der Größenordnung von 0,1 bis 10 Gew.-% und vorzugsweise 0,5 bis 5 Gew.-%.

5. Kosmetische Zusammensetzung nach einem der Ansprüche 1 bis 4, **dadurch gekennzeichnet, dass** sie weiterhin mindestens einen an Flavonoiden und/oder Tanninen reichen pflanzlichen Wirkstoff enthält, der ausgewählt ist aus der Gruppe, die von einem Auszug aus Wildkarde *(Dipscacus sylvestris)*, einem Auszug aus Rosmarin *(Rosmarinus officinalis),* einem Auszug aus Galgant *(Alpina officinarum)*, einem Auszug aus Schmetterlingsstrauch *(Buddleja davidii)* gebildet wird.

6. Kosmetische Zusammensetzung nach einem der vorangehenden Ansprüche, **dadurch gekennzeichnet, dass** sie weiterhin eine oder mehrere Formulierungssubstanzen oder Zusatzstoffe umfasst wie Weichmacher, Farbstoffe, filmbildende Mittel, Tenside, Duftstoffe, Konservierungsstoffe, Öle, Glycole, Vitamine und UV-Filter.

7. Verwendung einer kosmetischen Zusammensetzung nach einem der Ansprüche 1 bis 6, um der Hautalterung vorzubeugen und/oder um diese zu bekämpfen.

8. Verwendung von Auszügen aus *Diospyros kaki* und aus *Pueraria lobata*, wie in einem der Ansprüche 1 bis 4 definiert, für die Zubereitung einer kosmetischen oder dermatologischen Zusammensetzung, um der Hautalterung vorzubeugen und/oder um diese zu bekämpfen.
